# EUROPEAN PATENT APPLICATION

(11) **EP 4 174 187 A1**
(43) Date of publication of application: **03.05.2023**
(21) Application number: 20943447.1
(22) Date of filing: 31.12.2020
(51) Int. Cl.: C12P 21/08, C12M 3/00, C07K 16/46

(54) **METHOD FOR PREPARING BISPECIFIC ANTIBODY USING ANNULAR ORBITALLY SHAKEN BIOREACTOR**

(30) Priority: 30.06.2020 CN 202010620088
(71) Applicant: Guangzhou Lintonpharm Co., Ltd., Guangzhou, Guangdong 510320 (CN); Guangzhou Cantonbio Co., Ltd., Guangzhou, Guangdong 510320 (CN); Foshan Canton Biologics Co., Ltd., Foshan, Guangdong 528315 (CN); Progenbio Co., Ltd., Foshan, Guangdong 528312 (CN)
(72) Inventor: LU, Jinkang, Guangzhou, Guangdong 510320 (CN); JING, Tao, Guangzhou, Guangdong 510320 (CN); WU, Xiaobo, Guangzhou, Guangdong 510320 (CN); HU, Fei, Guangzhou, Guangdong 510320 (CN)
(74) Representative: Latscha Schöllhorn Partner AG
(86) International application number: PCT/CN2020/141964
(87) International publication number: WO 2022/001070

(57) **Abstract**

Disclosed is a method for preparing a bispecific antibody using an annular orbitally shaken bioreactor, which adjusts the rotational speed and the pH in cell culture conditions, and other culture conditions. It is found that the cell culture method of the present invention can maintain a high cell viability for a long time and prolong the culture time. Prolonging the culture time in an annular orbitally shaken reactor can obtain a higher cell density and a long-term high cell viability.

## Description

### Field of the Invention

The present invention relates to the field of biotechnology, and in particular to a method for preparing bispecific antibody using orbital shaken bioreactor.

### Background of the Invention

Antibodies have attracted increasing attention as medicaments due to their high stability and few side effects in blood. A bispecific antibody refers to an antibody that can simultaneously recognize two antigens (antigen A and antigen B). At present, bispecific antibodies are mainly expressed and produced in animal cells *in vitro.* Stirred bioreactors are conventional reactors in the field of cell culture. Although the technology is relatively mature, there are still many problems, which would bring about obstacles to the process of scaled up cell culture. First of all, in the process of scaled up cell culture, shear force originated from the stirring paddle causes damage to, or even death of, cells. Secondly, stirred bioreactors generally adopt a deep aeration mode, which would readily generate foam, and meanwhile, bubble rupture would cause even greater shear force. In addition, in the process of scaled up culture, stirred bioreactors would cause difficulty or even failure of scale-up due to various parameters and complex hydrodynamics involved.

Orbital shaken bioreactors are a kind of bioreactors for cell culture in a mode of being orbital shaken and mixed, and can well solve the problems existing with stirred bioreactors. However, there is a significant difference between the orbital shaken bioreactor and the stirred bioreactor in terms of working principle, such that the culture conditions in the stirred bioreactor are not suitable for the orbital shaken bioreactor. There has been no report in the prior art on a method for producing a bispecific antibody by culturing animal cells using an orbital shaken bioreactor. Therefore, it is an urgent problem to be solved to provide a method for producing a bispecific antibody by culturing animal cells using an orbital shaken bioreactor.

In the prior art, there are some defects in producing monoclonal antibodies by culturing animal cells using a stirred bioreactor. Firstly, in the process of scaled up culture, shear force originated from the stirring paddle causes damage to, and even death of, cells. Secondly, a stirred bioreactor generally adopts a deep aeration mode, which would readily generate foam, and meanwhile, bubble rupture would cause even greater shear force. In addition, in the process of scaled up culture, stirred bioreactors would cause difficulty or even failure of scale-up due to various parameters and complex hydrodynamics involved.

### Summary of the Invention

In order to solve the above problems, the present invention aims to provide a method for preparing bispecific antibody by using orbital shaken bioreactor.

The technical solution adopted by the present invention is as follows:
In one aspect of the present invention, provided is a method for preparing bispecific antibody by using orbital shaken bioreactor, comprising culturing cells in an orbital shaken bioreactor. The orbital shaken bioreactor has a rotational speed increasing at least once with the increase of number of days of culture, and the rotational speed is in the range from 85 rpm to 110 rpm. The inventors found that the cells can maintain a high cell viability at the rotational speed as described above.

According to an embodiment of the present invention, the rotational speed is increased by 2-3 rpm each time at supplementing, supplementing glucose or supplementing glutamine and is in the range from 85 rpm to 108 rpm. The rotational speed for cell culture is:
about 85 rpm from D0 to D1,
about 90 rpm from D2 to D3,
about 92 rpm at D4,
about 94 rpm at D5,
about 97 rpm at D6,
about 100 rpm at D7,
about 102 rpm at D8,
about 104 rpm from D9 to D10,
about 106 rpm from D11 to D12, and
about 108 rpm from D13 to the end of culture.

According to the embodiment of the present invention, a temperature for cell culture is in the range from 30 °C to 40 °C, and is decreased at D4 or D5.

According to the embodiment of the present invention, the temperature for cell culture is in the range from 35 °C to 40 °C before being decreased and in the range from 30 °C to 35 °C after being decreased.

According to the embodiment of the present invention, the temperature for cell culture is: about 37 °C from D0 to D4, and about 33 °C from D5 to the end of culture.

According to the embodiment of the present invention, a culture medium for cell culture comprises a basal medium, glucose, and glutamine.

According to the embodiment of the present invention, the basal medium is Excel-Advanced CHO Fed-batch culture medium purchased from Sigma Healthcare Limited.

According to the embodiment of the present invention, the feed medium is a Feed 4 culture medium, which is purchased from the Irvine Company LLC under the trade name of BalanCD CHO Feed 4.

According to the embodiment of the present invention, 0.8 × BalanCD CHO Feed 4 is used as a feed medium. The proportion of an added amount of the feed medium to the cell culture volume is: about 4% at D2, about 4% at D4, about 6% at D6, about 6% at D8, about 6% at D10, and about 6% at D12.

According to the embodiment of the present invention, the glucose has a concentration in the range from 4 g/L to 8g/L.

Furthermore, the glucose has a concentration in the range from 5 g/L to 8g/L.

According to the embodiment of the present invention, the glutamine has a concentration in the range from 2 g/L to 8g/L.

Furthermore, the glutamine has a concentration in the range from 4 mmol/L to 8 mmol/L from D0 to D4, and about 6 mmol/L from D5 to the end of culture.

During cell culture, the concentration of glucose and that of glutamine in the medium are monitored in real time. Glucose and glutamine are fed where the concentrations thereof are lower than the above ranges.

According to the embodiment of the present invention, the culture medium for cell culture has a pH value in the range from 6.8 to 7.2.

According to the embodiment of the present invention, the culture medium for cell culture has a pH value in the range from 6.9 to 7.0.

Furthermore, the pH value of the culture medium is:
about 7.0 from D0 to D2,
about 6.9 from D3 to D5, and
about 7.0 from D6 to the end of culture.

During cell culture, the pH value of the medium is monitored in real time. 1 M NaHCO₃ is added to increase the pH value where it is lower than the above range; and CO₂ is added to reduce the pH value where it is lower than the above pH value range.

According to the embodiment of the present invention, the bispecific antibody is anti-CD3 × anti-CD20 bispecific antibody.

According to the embodiment of the present invention, the rotational speed for cell culture can be increased once in the mid-term of culture. The rotational speed is in the range from 90 rpm to 100 rpm as follows: 90 rpm from D0 to D6 and 100 rpm from D7 to the end of culture.

According to the embodiment of the present invention, the temperature for cell culture is in the range from 30 °C to 40 °C, and is decreased at D4 or D5.

According to the embodiment of the present invention, the temperature for cell culture is in the range from 35 °C to 40 °C before being decreased and in the range from 30 °C to 35 °C after being decreased.

According to the embodiment of the present invention, the temperature for cell culture is: about 37 °C from D0 to D3, and about 31 °C from D4 to the end of culture. Such a temperature condition is more favorable for cell culture and for keeping a high cell viability.

According to the embodiment of the present invention, the culture medium for cell culture comprises a basal medium, glucose, and glutamine.

According to the embodiment of the present invention, the basal medium is Excel-Advanced CHO Fed-batch culture medium, which is purchased from Sigma Healthcare Limited.

According to the embodiment of the present invention, the feed medium 1 × BalanCD CHO Feed 4 is used. The proportion of added amount of the feed medium to the cell culture volume is: about 3% at D3, about 3% at D5, about 3% at D7, about 3% at D9, and about 3% at D11.

According to the embodiment of the present invention, the glucose has a concentration in the range from 4 g/L to 8g/L.

According to the embodiment of the present invention, the glutamine has a concentration is in the range from 2 mmol/L to 4 mmol/L from D0 to the end of culture.

During cell culture, the concentration of glucose and that of glutamine in the medium are monitored in real time. Glucose and glutamine are fed where the concentrations thereof are lower than the above ranges.

According to the embodiment of the present invention, the pH value of the medium for cell culture is: about 7.0 from D0 to the end of culture. The above pH value range is conducive to the generation of cellular nutrients.

During cell culture, the pH value of the medium is monitored in real time. 1 M NaHCO₃ is added to increase the pH value where it is lower than the above pH value range; and CO₂ is added to decrease the pH value where it is lower than the above pH value range.

According to the embodiment of the present invention, the bispecific antibody is anti-CD3 × Anti-HER2 bispecific antibody.

According to the embodiment of the present invention, the culture medium for cell culture has dissolved oxygen in the range from 20% to 80%. During cell culture, the dissolved oxygen in the culture medium is monitored in real time, and controlled within the above range by introduction of air.

According to the embodiment of the present invention, a ventilatory rate is in the range from 200 mL/min to 300 mL/min.

According to the embodiment of the present invention, an initial volume for cell culture is in the range from 6 L to 7.5 L.

According to the embodiment of the present invention, an initial cell seeding density is in the range from 0.3 × 10⁶ cells/mL to 0.5 × 10⁶ cells/mL.

According to the embodiment of the present invention, the cells are Chinese hamster ovary (CHO) cells.

According to the embodiment of the present invention, the Chinese hamster ovary (CHO) cell comprises nucleic acids encoding a bispecific antibody.

The beneficial effects of the present invention are as follows:
The cell culture method of the present invention can maintain long-term high cell viability and prolong the cell culture time.

Compared with the cell culture method using a stirred bioreactor, the cell culture method using the orbital shaken reactor of the present invention can obtain higher cell density and a long-term higher cell viability by prolonging the culture time. For some bispecific antibodies, the orbital shaken reactor results in higher protein expression levels.

### Brief Description of Drawings

FIG. 1 shows the viable cell density (VCD) and viability (VIA) in the cell cultures in Comparative Example 1, Comparative Example 2, and Example 1 of the present invention.
FIG. 2 shows the antibody titer in the cell cultures in Comparative Example 1, Comparative Example 2, and Example 1 of the present invention.
FIG. 3 shows the viable cell density (VCD) and viability (VIA) in the cell cultures in Comparative Example 3 and Example 2 of the present invention.
FIG. 4 shows the antibody titer in the cell cultures in Comparative Example 3 and Example 2 of the present invention.

### Detailed Description of Embodiments

The orbital shaken bioreactor used in Example 1 of the present invention was SB10-X (Kuhner) as an example. The bioreactor used in Comparative Example 1 was a 3L stirred bioreactor (My-control Applikon), and the bioreactor used in Comparative Example 2 was a 15L stirred bioreactor (EZ2-control Applikon).

The cell strains used in Example 1 of the present invention, Comparative Example 1, and Comparative Example 2 were Chinese hamster ovary (CHO) cells (CHO Express^{™}) expressing anti-CD3 × Anti-CD20 bispecific antibody.

The feed medium Feed 4 used in Examples 1-2 of the present invention and Comparative Examples 1-3 was purchased from the Irvine Company LLC under the trade name of BalanCD CHO Feed 4. The culture medium used in the present invention was purchased from Sigma Healthcare Limited under the trade name of Excell-Advanced CHO Fed-batch medium. The terms D0, D1, D2, D3, D4... used in the present invention represent day 0, day 1, day 2, day 3, day 4...

### Example 1

A method for culturing cells using an orbital shaken bioreactor, comprising: inoculating a cell strain in a culture medium according to a certain density for cell culture. The cell culture conditions of Comparative Example 1, Comparative Example 2, and Example 1 were shown in Table 1.

**Table 1 Culture conditions of Comparative Examples 1-2 and Example 1**

| Culture condition | Comparative Example 1 | Comparative Example 2 | Example 1 |
|---|---|---|---|
| Initial volume of cell culture (L) | 1.2 | 7.0 | 6.0 |
| Initial cell seeding density (10⁶ cells/mL) | 0.5 | 0.5 | 0.5 |
| Culture medium | Excell-Advanced CHO Fed-batch | | |
| Feed | 0.8 × BalanCD CHO Feed 4 | | |
| | Feed volume: the proportion of the feed volume to the cell culture volume was: 4% at D2, 4% at D4, 6% at D6, 6% at D8, 6% at D10, and 6% at D12. | | |
| Glucose (g/L) | In the range from 5 g/L to 8 g/L | | |
| Glutamine (mmol/L) | In the range from 4 mmol/L to 8 mmol/L from D0 to D4 6 mmol/L from D5 to end of culture | | |
| Rotational speed (rpm) | 300 from D0 to D4 | 190 from D0 to D4 | 85 from D0 to D1 |
| | | | 90 from D2 to D3 |
| | | | 92 at D4 |
| | | | 94 at D5 |
| | 350 from D5 to end of culture | 230 from D5 to end of culture | 97 at D6 |
| | | | 100 at D7 |
| | | | 102 at D8 |
| | | | 104 from D9 to D10 |
| | | | 106 from D11 to |
| | | | D12 |
| | | | 108 from D13 to end of culture |
| Temperature (°C) | 37 °C from D0 to D4 | | |
| | 33 °C from D5 to end of culture | | |
| Ventilatory rate (Surface vent-air) (mL/min) | 50 | 200 | 300 |
| Ventilatory rate (Bottom vent-air) (L/min) | 12 | 70 | N/A |
| pH value | 7.0±0.2 | 7.0±0.2 | 7.0 from D0 to D2 |
| | | | 6.9 from D3 to D5 |
| | | | 7.0 from D6 to end of culture |
| Dissolved oxygen (%) | 50% | 50% | 50% |

| | | | |
|---|---|---|---|
| N/A means not applicable. | | | |

pH value control strategy: the pH value of the culture medium was monitored in real time for Comparative Examples 1 and 2 and Example 1. 1 M NaHCO₃ was added to increase the pH value where it was lower than the above pH value range; and CO₂ was added to decrease the pH value where it was lower than the above pH value range.

DO control strategy: pure oxygen was added to increase dissolved oxygen in Comparative Examples 1 and 2, and air and oxygen were added to increase dissolved oxygen in Example 1.

In the process of culture, cell density and viability were detected by a cell counter (Beckman Coulter Inc.), and antibody titer was detected by Octet RED 96.

### 1. Viable cell density (VCD) and viability (VIA)

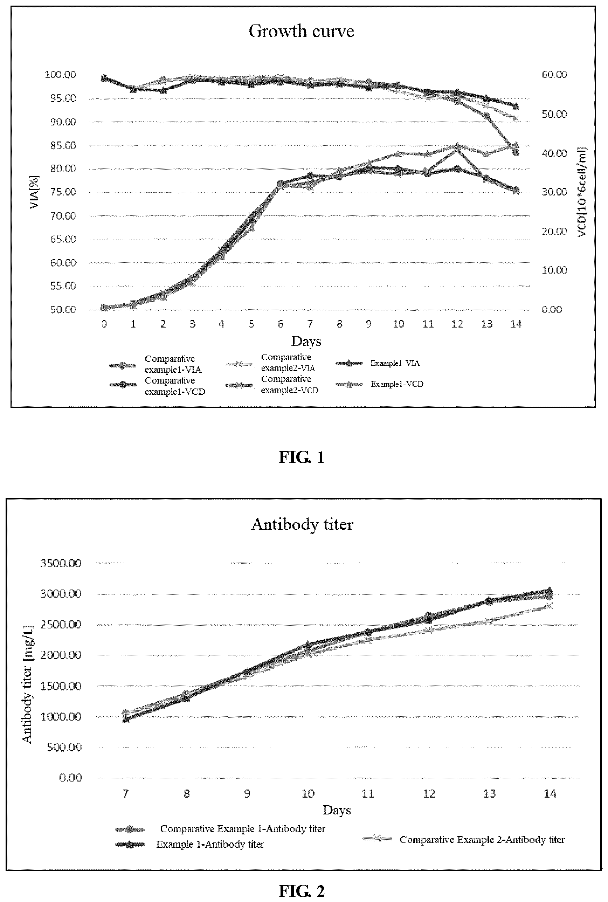

As shown in FIG. 1, the culture method in this Example could achieve a higher cell density and maintain long-term high cell viability compared with the conventional method using a stirred bioreactor (3 L, 15 L).

### 2. Antibody titer

Referring to FIG. 2 for the antibody titer, there was no significant difference in protein expression between the culture method in this Example and the method using a stirred bioreactor. The culture method in this Example not only maintained long-term high cell viability, but also had a higher yield of anti-CD3 × anti CD20 bispecific antibody.

The cell strains used in Example 2 and Comparative Example 3 of the present invention were Chinese hamster ovary (CHO) cells (CHO Express^{™}) expressing anti-CD3 × anti-HER2 bispecific antibody.

The orbital shaken bioreactor used in Example 2 of the present invention was SB10-X (Kuhner), and the stirring bioreactor used in Comparative Example 3 was a 3L stirring bioreactor (My-control Applikon).

The cell strains were cultured in the media with the culture conditions shown in Table 2:

**Table 2 Culture conditions for Comparative Example 3 and Example 2**

| Culture condition | Comparative Example 3 | Example 2 |
|---|---|---|
| Initial volume of cell culture (L) | 1.2 | 7.5 |
| Initial cell seeding density (10⁶ cells/mL) | 0.3 | 0.3 |
| Culture medium | Excellent-Advanced CHO Fed-batch | |
| Feed | 1 × BalanCD CHO Feed 4 | |
| | Feed volume: the proportion of the feed volume to the cell culture volume was: about 3% at D3, about 3% at D5, about 3% at D7, about 3% at D9, and about 3% at D11. | |
| Glucose (g/L) | In the range from 4 g/L to 8 g/L (adjusted with 45% glucose) | |
| Glutamine (mmol/L) | In the range from 2 mmol/L to 4 mmol/L | |
| Rotational speed (rpm) | 300 | 90 from D0 to D6 |
| | | 100 from D7 to end of culture |
| Temperature (°C) | 37 °C from D0 to D3 | |
| | 31 °C from D4 to end of culture | |
| Dissolved oxygen (%) | 50 | |
| pH value | 7.0±0.01 | 7.0 |
| Ventilatory rate (Surface vent-air) (mL/min) | 50 | 200 |
| Ventilatory rate (Bottom vent-air) (L/min) | 12 | N/A |

| | | |
|---|---|---|
| N/A: Not applicable. | | |

pH value control strategy: the pH value of the culture medium was monitored in real time for Comparative Example 3 and Example 2. 1 M NaHCO₃ was added to increase the pH value where it was lower than the above pH value range; and CO₂ was added to decrease the pH value where it was lower than the above pH value range.

DO control strategy: pure oxygen was added in Comparative Example 3 to increase dissolved oxygen, and air and oxygen were added in Example 2 to increase dissolved oxygen.

In the process of culture, cell density and viability were detected by a cell counter (Beckman Coulter Inc.), and antibody titer was detected by Octet RED 96.

### Detection results

### 1. Viable cell density (VCD) and viability (VIA)

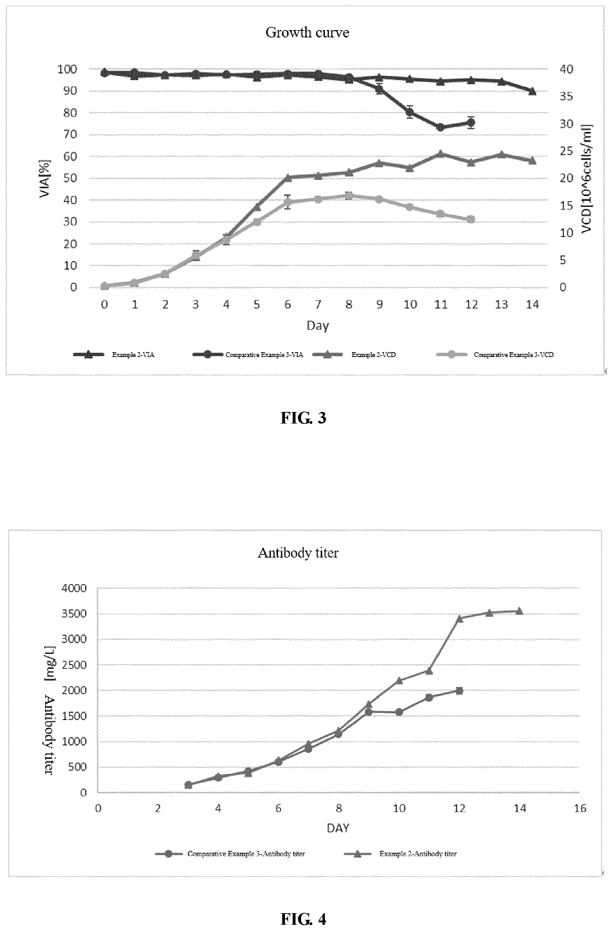

As can be seen from FIG. 3, the culture method in this Example was more suitable for expressing anti-CD3× anti-HER2 bispecific antibody than a conventional method using a stirred bioreactor. The culture method in this Example could obtain higher cell density and maintain higher cell viability.

### 2. Antibody titer

As can be seen from FIG. 4, the culture method in this Example could obtain a higher protein expression level, which could reach 3.5 g/L.

The above Examples are better embodiments of the present invention, but the embodiments of the present invention are not limited thereby. Any other changes, modifications, substitutions, combinations, and simplifications that do not deviate from the spirit and principle of the present invention should be equivalent replacement manners, and are included in the scope sought for protection by the present invention.

## Claims

1. A method for preparing bispecific antibody using orbital shaken bioreactor, comprising culturing cells in an orbital shaken bioreactor, wherein the orbital shaken bioreactor has a rotational speed increasing at least once with the increase of number of days of culture, and the rotational speed is in the range from 85 rpm to 110 rpm.

2. The method for preparing bispecific antibody using orbital shaken bioreactor according to claim 1, wherein a temperature for cell culture is in the range from 30 °C to 40 °C and the temperature for cell culture is decreased at D4 or D5.

3. The method for preparing bispecific antibody using orbital shaken bioreactor according to claim 2, wherein the temperature for cell culture is in the range from 35 °C to 40 °C before being decreased and in the range from 30 °C to 35 °C after being decreased.

4. The method for preparing bispecific antibody using orbital shaken bioreactor according to claim 1, wherein a medium for cell culture comprises a basal medium, glucose, and glutamine.

5. The method for preparing bispecific antibody using orbital shaken bioreactor according to claim 4, wherein:
the basal medium is Excel-Advanced CHO Fed-batch culture medium; and/or
a feed medium is Feed 4; and/or
the glucose has a concentration in the range from 4 g/L to 8 g/L; and/or
the glutamine has a concentration in the range from 2 mmol/L to 8 mmol/L.

6. The method for preparing bispecific antibody using orbital shaken bioreactor according to claim 5, wherein:
the culture medium for cell culture has a pH value in the range from 6.9 to 7.0;
and/or
the culture medium for cell culture has dissolved oxygen in the range from 20% to 80%.

7. The method for preparing bispecific antibody using orbital shaken bioreactor according to claim 1, wherein:
an initial volume for cell culture is in the range from 6 L to 7.5 L; and/or
an initial cell seeding density is in the range from 0.3 × 10⁶ cells/mL to 0.5 ×10⁶ cells/mL.

8. The method for preparing bispecific antibody using orbital shaken bioreactor according to any one of claims 1-7, wherein the cells are Chinese hamster ovary cells.

9. The method for preparing bispecific antibody using orbital shaken bioreactor according to claim 8, wherein the Chinese hamster ovary cells comprise nucleic acids encoding the bispecific antibody.

10. The method for preparing bispecific antibody using orbital shaken bioreactor according to claim 9, wherein the bispecific antibody is anti-CD3 × anti-CD20 bispecific antibody or anti-CD3 × anti-HER2 bispecific antibody.
